# EUROPEAN PATENT APPLICATION

(11) **EP 2 492 687 A1**
(43) Date of publication of application: **29.08.2012**
(21) Application number: 11001499.0
(22) Date of filing: 23.02.2011
(51) Int. Cl.: G01N 33/574

(54) **MYH10 as a new diagnostic marker of pathologies resulting from RUNX1 inactivation**

(71) Applicant: INSTITUT GUSTAVE ROUSSY -IGR-, 94805 Villejuif Cedex (FR)
(72) Inventor: Bluteau, Dominique, 75012 Paris (FR); Chang-Marchand, Yunchua, 13013 Paris (FR); Favier, Rémi, 92330 Sceaux (FR); Lordier, Larissa, 94800 Villejuif (FR); Raslova, Hana, 91830 Le Courdry Montceaux (FR); Vainchenker, William, 75005 Paris (FR)
(74) Representative: Barbot, Willy

(57) **Abstract**

The present invention relates to a method of diagnosing a pathology resulting from a runt-related transcription factor 1 (RUNX1) inactivation in a subject, which comprises the step of i) determining the platelets' myosin non-muscle heavy chain 10 (MYH10) expression level in a biological sample from said subject, and wherein a detectable platelets' MYH10 expression level is indicative of a pathology resulting from a runt-related transcription factor 1 (RUNX1) inactivation, and to a kit for diagnosing a pathology resulting from a runt-related transcription factor (RUNX1) inactivation in a subject comprising i) at least one antibody for determining the platelet MYH10 expression level in a biological sample from said subject, which can be used in a such a method.

## Description

### Field of the Invention

The present invention relates to a new marker to diagnose pathologies resulting from mutations in the *RUNX1* gene, such as FPD/AML, CMML and AML.

### Background

Hematopoiesis is maintained by a hierarchical system where hematopoietic stem cells (HSCs) give rise to multipotent progenitors, which in turn differentiate into all types of mature blood cells including platelets.

Said platelets are small anucleate cells that travel near the vessel wall during laminar flow. In response to vascular injury, platelets undergo alterations in morphology, which allow them to aggregate and cover the injured site. Platelets are produced during megakaryopoiesis in a process that involves the formation of platelet precursors called proplatelets and subsequent release of these proplatelets into the circulation. By forming a demarcation membrane system within the cytosol, megakaryocytes contain a membrane reservoir, which allows for the production of thousands of platelets per mature megakaryocyte. This complex differentiation process called megakaryopoiesis is under the control of several regulators, the main one being thrombopoietin (TPO). TPO is necessary for megakaryocyte maturation in that TPO deficient mice display greatly reduced megakaryocyte production as well as reduced numbers of mature megakaryocytes. Several transcription factors have also been implicated in megakaryopoiesis including, GATA-1, friend of GATA-1 (FOG-1), nuclear factor-erythroid 2 (NF-E2), runt-related transcription factor 1 (RUNX1) and Fli-1.

RUNX1, also known as AML1, CBFA2, AMLCR1, or PEBP2aB has been associated with several human pathologies including FPD/AML, Acute Myeloid Leukemia (AML), and also Myelodysplastic/Myeloproliferative disorders (i.e. chronic myelomonocytic leukemia (CMML)). The implication of RUNX1 in these pathologies results from mutations in the *RUNX1* gene, such as non sense mutations resulting in frame shifts or translocations resulting in chimeric fusion proteins (Blyth et al., Nat Rev Cancer vol.5, p: 376-3871, 2005).

Familial platelet disorder with propensity to acute myeloid leukaemia (FPD/AML; OMIM 601399) is an autosomal dominant disorder in which affected members have a clinical history of bleeding tendency and mild to moderate thrombocytopenia with normal platelet size and morphology, and/or abnormal platelet aggregation in response to arachidonic acid (HO et al., Blood, vol.87, p: 5218-5224, 1996). FPD/AML has been associated with RUNX1 (Song et al., Nat Genet, vol.23, p: 166-175, 1999; Michaud et al., Blood, vol.99, p :1364-1372,2002)

Acute Myeloid leukaemia (AML) are usually according to the FAB classification on their morphology from M1 to M7. They can be classified also on other criteria based essentially on genetic abnormalities for example in four groups: (i) the AML with recurrent genetic abnormalities AML t(8;21)(q22;q22) with RUNX1-ETO fusion gene (Peterson et al., Oncogene, vol.23, p :4255-62, 2004); AML with abnormal bone marrow eosinophils and inv(16)(p13;q22) or t(16;16)(p13;q22) with CBF□/MYH11 rearrangement; acute promyelocytic leukaemia APL with t(15;17)(q22;q12) PML/RARA; AML with 11q23 (MLL) abnormalities); (ii) AML with multilineage dysplasia following MDS or MDS/MPD or without antecedent of MDS or MPD; (iii) AML or MDS therapy related and (iv) other unclassified AML among that comprises the group of AML with normal karyotype which prognosis is based on molecular analysis of oncogenes such as mutations of FLT3-ITD or NPM1.

Myelodysplastic/myeloproliferative syndromes include four myeloid diseases grouped in 1999 by the WHO: chronic myelomonocytic leukemia (CMML), juvenile myelomonocytic leukemia (JMML), atypical chronic myeloid leukemia (aCML) and unclassified myelodysplastic/myeloproliferative syndromes (U-MDS/MPS). Chronic myelomonocytic leukemia (CMML) has 4 defining features including an absolute monocytosis of >1x10⁹/1, the absence of Philadelphia chromosome or BCR-ABL fusion gene or a fusion gene that include *PDGFRb* gene (e.g. TEL-PDGFRb fusion gene), a percentage of blast cells in the bone marrow lower than 20%, and a variable degree of dysplasia in all three lineages. Myeloblasts and promonocytes comprise less than 5% of nucleated cells in peripheral blood. Roughly half of patients present with an elevated white cell count that is commonly associated with hepatomegaly and splenomegaly, the so-called myeloproliferative form of the disease (Kuo et al., Leukemia, vol.23, p: 1426-31, 2009).

In the diagnostic of these pathologies, mostly for FPD/AML or in the understanding of their heterogeneity (CMML), it is necessary to sequence the complete *RUNX1* gene sequence. Nevertheless, and since FPD/AML is very rare, this sequencing is done in a very large excess resulting in an important cost. In addition many FPD/AML are undiagnosed because due to their rarity, RUNX1 is not sequenced systematically in familial thrombocytopenia.

### Summary of the Invention

The present inventors now discover that the expression of MYH10 is repressed by RUNX1 during megakaryopoiesis. The inventors have further established that in RUNX1 knock-out mice, MYH10 expression is detected in mature megakaryocytes, whereas said MYH10 protein is not detected in wild type mice.

Finally, the inventors provide with the detection of MYH 10 expression in platelets, a new biomarker indicative of a pathology associated with RUNX1 inactivation.

Thus, a first object of the invention relates to a method of diagnosing a pathology resulting from a runt-related transcription factor 1 (RUNX1) inactivation in a subject, which comprises the step of i) determining the platelets' MYH10 expression level in a biological sample from said subject, and wherein a detectable platelets' MYH10 expression level is indicative of a pathology resulting from a runt-related transcription factor 1 (RUNX1) inactivation.

Advantageously, said pathology resulting from a runt-related transcription factor 1 (RUNX1) is selected in the group comprising familial platelet disorder with propensity to acute myeloid leukaemia (FPD/AML), acute myeloid leukaemia (AML) and chronic myelomonocytic leukemia (CMML), and preferably said pathology is FPD/AML.

Still advantageously, said method is for testing a subject thought to develop or to be predisposed to developing familial platelet disorder with propensity to acute myeloid leukaemia (FPD/AML), to acute myeloid leukaemia (AML) and/or to chronic myelomonocytic leukemia (CMML).

In a second object, the present invention relates to a kit for diagnosing a pathology resulting from a runt-related transcription factor 1 (RUNX1) inactivation in a subject, which comprises i) at least one antibody for determining the platelet MYH10 expression level in a biological sample from said subject.

Preferably, said kit further comprises ii) at least one mean for purifying platelets from the biological sample.

### Brief Description of the Drawings

Figure 1 shows the transcript expression level of MYH9 and MYH10 during MK differentiation.

Figure 2 shows the protein expression level of MYH9 and MYH10 during MK differentiation.

Figure 3 shows the ploidy level of MK from *myh9*^{*-*/*-*} mice as compared to that of wt MK.

Figure 4 shows the the ploidy level of MK from *myh9*^{*-*/*-*} mice as compared to that of wt MK in the presence of 50 uM of blebbistatin, the inhibitor of non-muscular myosins II.

Figure 5 shows the structure of the MYH10 gene and the relative occupancy of the RUNX1 binding site in the *MYH10* promoter by RUNX1 (at left) and by IgG as control (at right).

Figure 6 shows the two different used *MYH10* promoter-luciferase reporter gene constructs and the luciferase expression obtained with both constructions in the presence or absence of RUNX1 and/or of CBFβ.

Figure 7 shows the effect of RUNX1 knockdown on MYH10 expression.

Figure 8 shows the expression level of MYH10 in wt and in *Runx1* KO mice.

Figure 9 shows the MYH10 expression as compared to HSC70 in FPD/AML patients with Runx1 mutations (AII-1 AII-2, BII-2, BIII-1, and D) as compared to healthy individuals (without RUNX1 alteration) (C1, C2 and C3).

Figure 10 shows the Western blot analysis of MYH10 in platelets of CMML (1-7) patients and of one healthy individual used as a control.

### Detailed Description

In one aspect the present invention relates to a method of diagnosing a pathology resulting from a runt-related transcription factor 1 (RUNX1) inactivation in a subject, which comprises the step of i) determining the platelets' MYH10 expression level in a biological sample from said subject, and wherein a detectable platelets' MYH10 expression level is indicative of a pathology resulting from a runt-related transcription factor 1 (RUNX1) inactivation.

The *MYH10* (myosin non-muscle heavy chain 10, Gene ID: 4628) gene, which is also known as NMMHCB, MGC 134913, or MGC 134914, encodes for the myosin non-muscle heavy chain 10. MYH10 has the 1976 amino acid sequence SEQ ID N°1 (Accession number: NP_005955) and is encoded by the nucleic acid sequence SEQ ID N°2 (Accession number: NM_005964).

As used herein, the term "subject" refers to a mammal, preferably a human.

Pathologies resulting from a runt-related transcription factor (RUNX1) inactivation in a subject are well known from the skilled person and include familial platelet disorder with propensity to acute myeloid leukaemia (FPD/AML), acute myeloid leukaemia (AML) and/or chronic myelomonocytic leukemia (CMML).

As used herein an inactivation of runt-related transcription factor 1 (RUNX1) corresponds to a loss of RUNX1 DNA binding activity and/or to a loss RUNX1 transactivation activity (Harada et al., Blood, vol.103, p: 2316-24, 2004). Such inactivation of RUNX1 results from missense mutations in the *RUNX1* gene, like the mutations L56S and K83E in reference to the wild-type RUNX1 protein (SEQ ID n°3) or those disclosed in TANG et al. (Blood, vo1.114, p: 5352-61, 2009) or from translocations implying the *RUNX1* gene, like the translocations t(8;21), t(12;21) and t(3;21) which involves AML1-ETO, AML1-ETV6, AML1-MDS1 respectively (ROUMIER et al., Leukemia, vo1.17, p: 9-16, 2003).

Biological samples comprising platelets are well known from the skilled person and include blood samples and bone marrow samples. Preferably, the biological sample is a blood sample.

Methods for purifying platelets from biological samples such as blood samples are well known from the skilled person and are disclosed, as an example, in NIUL et al. (Comparative Haematology International, vol.2(2), p: 106-110, 1992), GNATENKO et al. (Methods Mol. Biol., vol.496, p:245-72, 2009), in SCHEDEL & ROLF (Methods Mol. Biol., vol.496, p:273-83, 2009), or in CHOI et al. (Lab. Chip., "Hydrophoretic high-throughput selection of platelets in physiological shear-stress range", 2010).

The subject may be healthy, but the method of the invention is particularly useful for testing a subject thought to develop or to be predisposed to developing familial platelet disorder with propensity to acute myeloid leukaemia (FPD/AML), acute myeloid leukaemia (AML) and/or chronic myelomonocytic leukemia (CMML). In that case, the method of the invention enables to confirm that said subject develops or is predisposed for developing FPD/AML, AML and/or CMML.

In a preferred embodiment, the method of the invention further comprises the step of(ii) comparing said platelets' MYH10 expression level in said biological sample with a control.

As used herein said "control" refers to the MYH10 expression level in a control sample corresponding to platelets of a biological sample from a healthy subject. Said control is preferably the average expression level of MYH10 in several control biological samples. As determined by the inventors, there is no detectable expression level of MYH10 in the platelets of healthy subjects.

Methods for determining the MYH10 expression level expression of MYH10 protein in platelets are well known for the man skilled in the art.

In another particular embodiment, the expression of MYH 10 is assessed by determining the level of expression of the MYH10 protein (i.e. SEQ ID N°1).

Such analysis can be assessed using an antibody (e.g., a radio-labeled, chromophore-labeled, fluorophore-labeled, or enzyme-labeled antibody), an antibody derivative (e.g., an antibody conjugate with a substrate or with the protein or ligand of a protein of a protein/ligand pair (e.g., biotin-streptavidin)), or an antibody fragment (e.g., a single-chain antibody, an isolated antibody hypervariable domain, *etc*.) which binds specifically to the MYH10 protein. Said analysis can be assessed by a variety of techniques well known by one of skill in the art including, but not limited to, enzyme immunoassay (EIA), radioimmunoassay (RIA), Western blot analysis and enzyme linked immunoabsorbant assay (ELISA).

Polyclonal antibodies can be prepared by immunizing a suitable animal, such as mouse, rabbit or goat, with the MYH10 protein (SEQ ID NO:1) or a fragment thereof (e.g., at least 10 or 15 amino acids). The antibody titer in the immunized animal can be monitored over time by standard techniques, such as with an ELISA using immobilized polypeptide. Such MYH10 polyclonal antibodies are already commercially available from SANTA CRUZ BIOTECHNOLOGY, INC., LIFESPAN BIOSCIENCES, AVIVA SYSTEMS BIOLOGY, or from SIGMA-ALDRICH.

At an appropriate time after immunization, e.g., when the specific antibody titers are highest, antibody producing cells can be obtained from the animal and used to prepare monoclonal antibodies (mAb) by standard techniques, such as the hybridoma technique originally described by KOHLER and MILSTEIN (Nature, vol.256, p:495-497, 1975), the human B cell hybridoma technique (KOZBOR et al., Immunol., vol.4, p: 72, 1983), the EBV- hybridoma technique (COLE et al., In Monoclonal Antibodies and Cancer Therapy, Alan R. Liss,Inc., p: 77-96, 1985) or trioma techniques. The technology for producing hybridomas is well known (see generally Current Protocols in Immunology, COLIGAN et al. ed. , John Wiley & Sons, New York, 1994). Hybridoma cells producing the desired monoclonal antibody are detected by screening the hybridoma culture supernatants for antibodies that bind the polypeptide of interest, e.g., using a standard ELISA.

In a second aspect, the present invention refers to a kit for diagnosing a pathology resulting from a runt-related transcription factor 1 (RUNX1) inactivation in a subject, which comprises i) at least one antibody, which can be used in a method as disclosed previously, for determining the platelet MYH10 expression level.

The kit of the invention can further comprise at least one mean for purifying platelets from a biological sample, preferably from a blood sample.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kits can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kits include nucleotide polymerases and the like. Solid supports can include beads and the like whereas molecular weight markers can include conjugatable markers, for example biotin and streptavidin or the like.

In one embodiment, the kit is made up of instructions for carrying out the method described herein. The instructions can be provided in any intelligible form through a tangible medium, such as printed on paper, computer readable media, or the like.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### 1) Identification a new non-muscle myosin II heavy chain expression during megakaryocyte differentiation

Hematopoietic progenitors were cultured in presence of thrombopoietin (TPO) and the CD41 positive cells were sorted at day 6, 9 and 12 of culture. mRNA was isolated with the RNEASY MINI or MICRO kit (QIAGEN) and cDNA was generated by reverse transcription (INVITROGEN). The expression of MYH10 and MYH9 was analyzed during MK differentiation by qRT-PCR and by western blot.

The qRT-PCR analysis was realized with probes or SYBR® green (APPLIED BIOSYSTEMS) on a PRISM® 7700 (APPLIED BIOSYSTEMS). Gene expression level of MYH10 and MYH9 was normalized to HPRT.

For the western blot analysis, the protein expression level of MYH10 and MYH9 was normalized to HSC70. The proteins were extracted from megakarocytes at day 6, 9 and 12 of culture in presence of TPO, from platelets of healthy control and from Hela cell line. The western blot was performed as previously described (GILLES *et al, Blood,* 2008) using the antibody directed against MYH9 (CELL SIGNALING), MYH10 (CELL SIGNALING) , and HSC70 (CTRESSGEN).

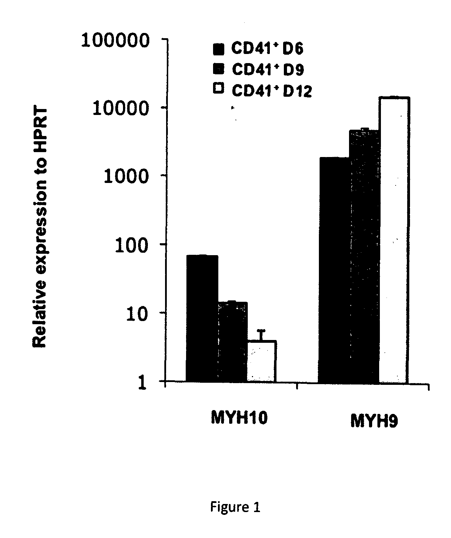

The figure 1 shows the transcript expression level of MYH9 and MYH10 during MK differentiation (CD41 cells at days 6, 9 and 12 of differentiation).

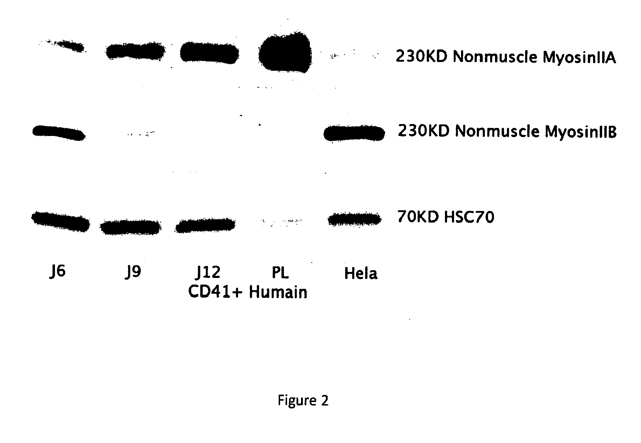

The figure 2 shows the protein expression level of MYH9 and MYH10 during MK differentiation (CD41 cells at days 6, 9 and 12 of differentiation).

The results show the expression of MYH 10 at the early stage of megakaryocyte (MK) differentiation, an expression decrease during MK maturation and polyploidization, whereas no MYH10 expression was detected in mature MK and platelets (Figures 1 and 2).

In contrast, the expression established that the expression level of another non-muscular myosin, MYH9, increases during MK maturation and in platelets (Figures 1 and 2).

### 2) Myosin II is activated to some extent in the contractile ring of mitotic/endomitotic MKs and inhibition of myosin II activity decreased actin turn over in MK contractile ring

The myosin II activation is controlled by phosphorylation of its regulatory light chain (MLC2). One antibody recognizing Ser¹⁹ and Thr¹⁸ phosphorylated MLC2 (PPMLC2) was used to examine myosin II activation at late telophase of mitosis and endomitosis.

The results have established that for a total of 102 dipolar mitotic/endomitotic MKs that were examined at late telophase (cleavage furrow ≥75% cell transversal diameter), a PPMLC2 positive signal was seen in 68.6% cells (data not shown, three independent experiments). In parallel, 24 multipolar endomitotic MKs were examined at late telophase or during the retro-cytokinesis process, and only 6 of them exhibited a positive signal for PPMLC2 localized either on all the parts of the cleavage furrow or on a part of it. These data suggest that myosin II is activated at some extent in the contractile ring during MK dipolar mitosis/endomitosis and this activation becomes less important during multipolar endomitosis.

Thus, it was shown that the achievement of cytokinesis required highly actin dynamics (assembly and disassembly) in the contractile ring and this dynamic behavior is regulated by myosin II activity.

To check if the contractile ring in MK does not works well due to a decline of myosin II activity, CD34⁺ cells were induced to MK or non MK differentiation by a cocktail of growth factors and transfected by the GFP-β-actin construct. CD41⁺ GFP⁺ and CD41⁻ GFP⁺ cells were sorted and the actin turnover was measured in the contractile ring at the end of cytokinesis by FRAP in the dipolar mitosis or endomitosis.

The results have shown that for MKs overexpressing actin-GFP, 55.7%±12.8 of the total fluorescence prior to bleach was recovered in the contractile ring, with a t_{1/2} of 6.3±2.5 sec (n=15, three independent experiments). In the contractile ring of non MK cells, 77.6%±10.9 of the total fluorescence prior to bleach was recovered with a t_{1/2} of 8.6±3.7 sec (n=16, three independent experiments). In contrast, similar rates of turnover were measured between MKs (50% total recovered fluorescence / t_{1/2} = 4.42%) or non MK cells (50% total recovered fluorescence / t_{1/2} = 4.40%). The finding that the actin-GFP fluorescence recovery was lower in the contractile ring of MKs (∼28%, p<0.0007) than in control myeloid cells suggests the existence of a higher pool of non dynamic F-actin at the end of cytokinesis of dipolar mitotic/endomitotic MKs (data not shown).

Interestingly, when we partially inhibited myosin II activity in CD41⁺ cells with a low concentration of Blebbistatin (12.5 µM), the fraction of nondynamic F-actin still increased in the contractile ring. Indeed, the actin-GFP fluorescence recovery in the contractile ring of MKs with Blebbistatin decreased from 55.7%±12.8 to 42.9%±9.0%, with t_{1/2} of 2.94±1.35 sec (n=8, p=0.01) (data not shown).

Together, these results suggested there still persisted a myosin II activity in the contractile ring of MKs; however, the attenuation of myosin II activity in the contractile ring increased the pool of non-dynamic F-actin accumulates at the end of cytokinesis, which increased the probability that a failure of cytokinesis occurs in MK.

### 3) Inhibition of Myosin II by Blebbistatin cancels furrow ingression and increases MK polyploidisation

To investigate the role of myosin II in MK endomitosis, Blebbistatin was used to inhibit its actin during megakaryocytosis. Blebbistatin is added into MK culture at day 6. The ploidy level of CD41⁺/CD42⁺ MKs was analyzed by flow cytometry 72h after addition of Blebbistatin.

The results have shown that the addition of Blebbistatin to MK cultures led to a significant increase in MK polyploidization because the mean ploidy was increased from 6.28N to 12.13N (100µM Blebbistatin, n = 4, p=0.008) and from 6.47N to 11.27N (25 µM, n=3, p=0.015; data not shown). This result suggests that activation of myosin II may play a negative role on MK polyploidization.

To check more precisely how myosin II inhibition affects the endomitosis process, we used real-time video microscopy to monitor endomitosis in H2B-GFP-expressing MKs treated with Blebbistatin (25 µM). Endomitotic MK treated with Blebbistatin or without Blebbistatin were filmed in 3 independent experiments.

The results have shown that Blebbistatin-treated MKs have an apparently normal metaphase-to-anaphase transition (data not shown). However, compared to the controls MK that show a late failure of cytokinesis, the furrow ingression of MKs treated by Blebbistatin was almost completely inhibited.

### 4) MK polyploidisation is not changed in mice with megakaryocyte-restricted MYH9 inactivation but it is increased by inhibition of mysin II by Blebbistatin

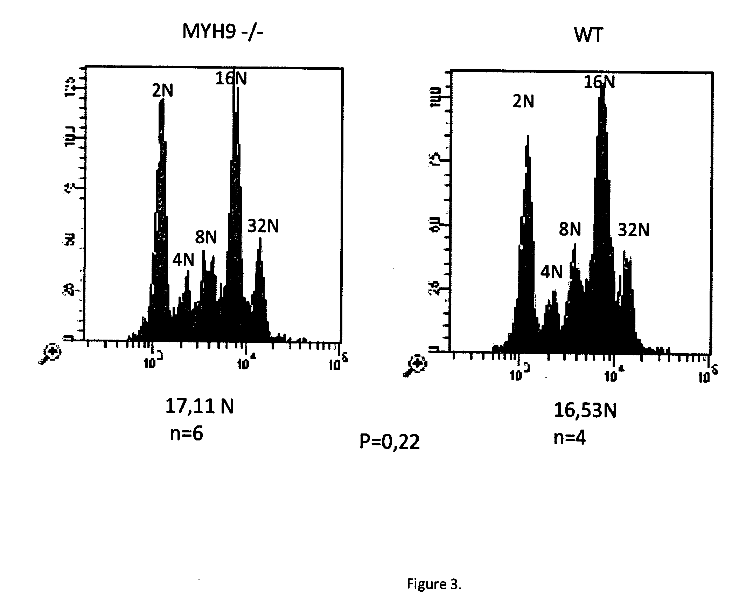

We demonstrated that MYH9 is not involved in the process of MK ploidization as the ploidy level of MK from *myh9*^{*-*/*-*} mice is not different from that of wt MK (Figure 3).

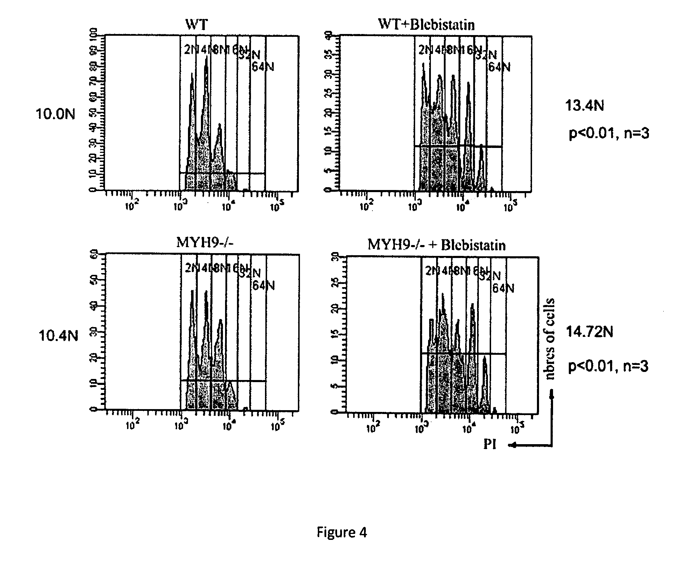

However, the presence of 50 µM of blebbistatin, the inhibitor of non-muscular myosins II leaded to an increase in mean ploidy level of *myh9*^{*-*/*-*} mice (from 10.40 to 14.72, p=0.01,n=3) as well as in wt control (from 10.00N to 13.40N, p=0.01,n=3) (Figure 4).

Together these results suggest that another myosin II than MYH9 is involved in the process of ploidization. As we demonstrated the presence of MYH10 in the early stages of megakaryopoiesis, we postulated that MYH10 could be the principal actor involved in the ploidization process.

Thus, the ploidy level of CD41⁺ MK was analyzed directly after dissociation the fetal liver as previously described for bone marrow MK (GILLES et al, Blood, 2009). Briefly, the hematopoietic progenitors (Lineage negative, Lin-, cells) were purified from fetal liver of E13.5 by the kit MILTENYI BIOTEC and were cultivated in serum free medium with TPO and stem cell factor (SCF). Blebbistatin was added in the culture just after the purification and ploidy level of CD41⁺ cells was analyzed at day 3 of culture.

### 5) Knock down of MYH10 by shRNA decreases MK proliferation and increases MK polyploidisation

Non-muscle myosin II heavy chain is one principal composition of contractile ring, which appeared at the beginning of telophase in the midzone.

To check if MYH10 is recruited normally in the contractile ring of mitotic and endomitotic MK, immuno-fluorescence was used to investigate CD41⁺ MK at day 6 of culture.

The results have shown that only one part of MK in telophase accumulated MYH10 in contractile ring (data not shown). The percentage of positive cells increased following the progression of telophase. At the 2N and 4N transition, MYH10 started to accumulate at the midzone in certain cells when the cleavage furrow appeared. When the depth of cleavage furrow is ≤75% cell transversal diameter, only 24.5% cells showed MYH10 accumulation (3 independents experiments and 85 telophase cells were counted) around midzone. However, at late telophase (cleavage furrow ≥75%), 57% cells show MYH10 accumulation at the cleavage furrow (3 independents experiments and 141 late telophase cells were counted). For multipolar endomitotic MKs (4N to 8N or >8N), only 9% cells had an incomplete MYH 10 accumulation at the cleavage furrow when it is ≤75% transversal diameter (3 independents experiments and 42 telophase cells were counted). The percentage is increased to 29% at the end of telophase (cleavage furrow ≥75% transversal diameter, 3 independents experiments and 17 late telophase cells were counted). These results suggest that MYH10 is implicated in the formation of contractile ring.

All the above results suggested that MYH10, but not MYH9 plays a negative role in endomitosis process. However, since inhibitors may lack specificity, we used more specific inhibition approaches by employing shRNA against MYH10 vectorized in a HIV-derived lentivirus.

CD34⁺ cells were infected at day 1 and day 2 of culture and the ploidy level of GFP⁺/CD41⁺ cells was analyzed by flow cytometry at day 9 of culture. The shRNA of MYH10 increased significantly MK ploidy (data not shown). The mean ploidy level was increased from 7.65N (control) to 9.84N (n = 3, p=0.015) in GFP⁺⁺ MK. In order to confirm that MYH9 or MYH10 was efficiently depleted by the shRNA, the GFP⁺ cell population was sorted 48 h after infection. Western, blot analysis revealed an about 40% reduction in the MYH10 protein level in comparison to the scramble (SCR) shRNA used as a control.

### 6) Runx1 regulates negatively MYH10 transcription during megakaryocyte differentiation

Previously we demonstrated that RUNX1, the master hematopoietic transcription factor, directly regulates transcription of the myosin light chain 9 (MYL9/MLC2) involved in proplatelet formation (GILLES et al Blood, 2009) and p19^{INK4D} involved in the arrest of endomitosis *(*GILLES et al, Blood, 2008). Small diploid megakaryocytes are present in the bone marrow of *runx1* KO mice. Thus, we hypothesized that RUNX1, already implicated in the ploidisation arrest via regulation of p19^{INK4D}, could be also involved in the regulation of endomitosis by directly targeting MYH10.

Using an *in silico* approach with the CHIPMAPPER online software, we identified one putative RUNX1 transcription factor-binding sites in the *MYH10* promoter region suggesting a direct regulation of its expression by RUNX1. To demonstrate that RUNX1 binds to the *MYH10* promoter *in vivo,* we conducted ChIP assays and showed that RUNX1 binds this site in MK

Chromatin immunoprecipitation assay were performed in megakaryocyte cells (9 days in culture with TPO) with primer sets (MYH10_A and _B) directed toward RUNX1-binding site predicted *in silico,* as previously described *(*GILLES et al, Blood, 2008).

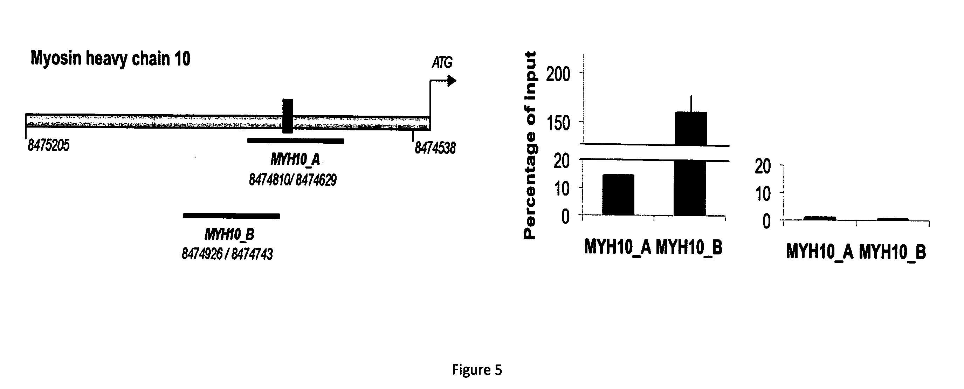

The figure 5 shows the structure of the MYH10 gene and the relative occupancy of the RUNX1 binding site in the *MYH10* promoter by RUNX1 (at left) and by IgG as control (at right). Error bars represent the standard deviation of two experiments.

To test for the functional relevance of this RUNX1 site, Promoter activity assays were performed as previously described (Gilles L et al, Blood 2008).

Briefly, luciferase assay was performed by transient transfection of HEL cells with MPI vector containing wild type RUNX1 cDNA and pEF6/V5-His-TOPO vector containing CBFβ cDNA. Relative luciferase activities are shown after normalization of inducible firefly luciferase activity relative to constitutive Renilla luciferase activity. Normalized expression was calculated relative to luciferase activity cells transfected with *MYH10* promoter-luciferase reporter gene construct alone. We used two different *MYH10* promoter-luciferase reporter gene constructs: pMYH10-luc and pMYH10mut_luc with mutated RUNX1 binding site.

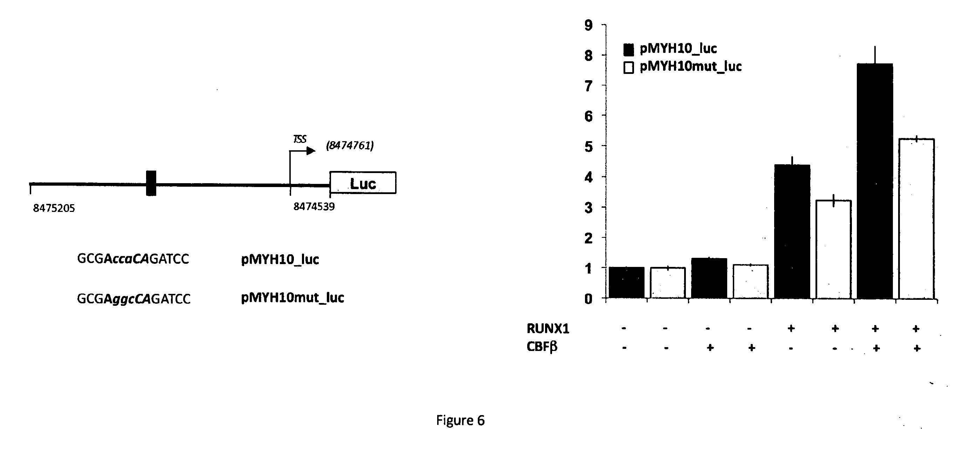

The figure 6 shows the two different used *MYH10* promoter-luciferase reporter gene constructs: pMYH10-luc (black) and pMYH10mut_luc (gray) with mutated RUNX1 binding site. The figure 6 also shows the luciferase expression obtained with both constructions in the presence or absence of RUNX1 and/or of CBFβ. The histograms show one representative experiment of two, each in triplicate. Error bars represent the standard deviation of triplicate. P<0.05.

The results established that the over-expression of wt RUNX1 with its cofactor CBFβ increased luciferase activity in transient transfection assays in HEL cells whereas this last one was significantly decreased after directed mutagenesis of the RUNX1 binding site (Figure 6).

These data strongly support that MYH10 is a direct transcriptional target of RUNX1.

### 7) Runx 1 Knock out increases MYH10 expression and decreased MK polyploidisation in vitro and in vivo

To confirm that MYH10 is a functional negative RUNX1 target in human megakaryocytes, we knocked down RUNX1 in CD34⁺ cells with a recently published shRNA of RUNX1 (shRUNX1) *(*GILLES et al, Blood, 2008).

Cells were than cultured in presence of thrombopoietin to obtain megakaryocytes. As shown previously, shRNA treatment led to a significant decrease (50%) in RUNX1 transcripts and thus mimics a RUNX1 haploinsufficiency.

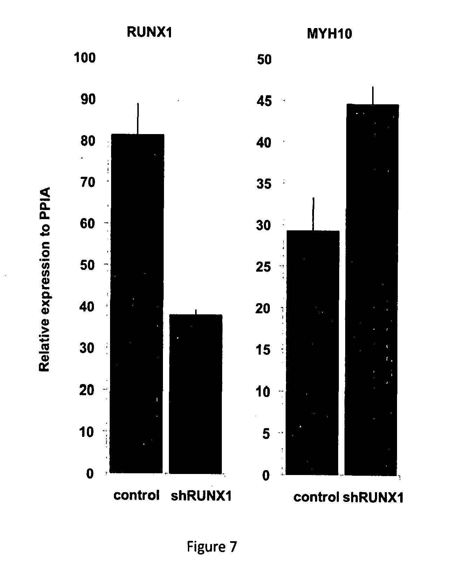

The figure 7 shows the effect of RUNX1 knockdown on MYH10 expression. Control CD34⁺ cells were transduced with the lentivirus encoding the scramble (control) shRNA or RUNX1 shRNA (shRUNX1). At day 6 of culture in presence of TPO alone, cells were sorted and RUNX1 and MYH10 levels were analyzed in CD41⁺42⁺GFP⁺ cells by Q-PCR. Their expression level was normalized to PPIA expression

The results show that in shRNAs-treated CD41⁺CD42⁺GFP⁺ cells (MK), a significant 1.5 fold increase in MYH10 expression was observed suggesting that MYH10 was negatively regulated by RUNX1 in MK (Figure 7).

The effect of RUNX1 knockdown on ploidy level of CD41⁺ MK was also determined. Control CD34⁺ cells were transduced with the lentivirus encoding the scramble (control, SCR) shRNA or RUNX1 shRNA (shRUNX1) and cultured in presence of TPO and SCF. The ploidy level was analyzed at day 9 of culture as previously described (GILLES et al, Blood, 2008).

The effect of Runx1 knock-out on MYH10 expression and on ploidy level was determined in *in vivo* model. The conditional *Runx1* mouse strain was developed by GROWNEY et al. (2005, Blood). These mice were crossed with mice Mx1-Cre mice and the deletion of *Runx1* locus flanked by LoxP sites was induced by injection of interferon-inducing agent pIpC. (Mx1 is the interferon inducible promoter and Cre is Cre-recombinase allowing the deletion of LoxP flanked sites). Five weeks after induction of *Runx1* KO, the platelet count was performed. The *Runx1* KO mice exhibited signs of thrombopenia (2-3x10⁵ platelets/ml) contrary to the mice negative for Cre-recombinase (about 1x10⁶ platelets/ml). The excision by Cre-recombinase of *Runx1* locus flanked by LoxP sites was verified by PCR on DNA isolated from bone marrow progenitors (Lin- cells). Lin- cells were cultured as described above in serum free medium in presence of TPO and SCF. At day 2.5, CD41⁺ cells were sorted and MYH10 expression level was analyzed by qRT-PCR.

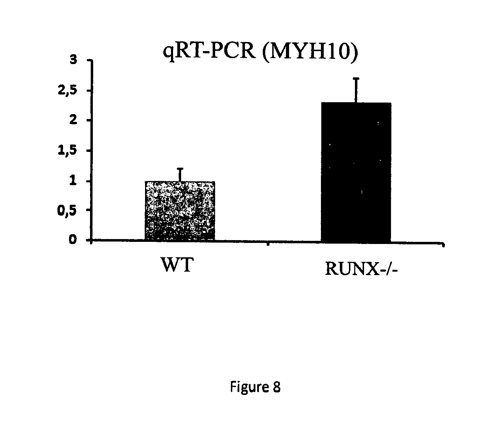

The Figure 8 shows the expression level of MYH10 in wt and in *Runx1* KO mice.

The results show that the MYH10 expression level was increased relatively to HPRT expression level in *Runx1* KO.

The effect of *Runx1* KO on ploidy level was analyzed directly on bone marrow MK as described by Gilles et al, (2008, Blood).

The results have shown that the RUNX1 knock-down and the increase in MYH10 led to the decrease in ploidy level of MK (data not shown). These results were confirmed also in an *in vivo* model. Indeed, as it was previously postulated, we confirmed that megakaryocytes of KO RUNX1 mice exhibited a lower ploidy level than MK of wt mice with an increased level of MYH10.

### 8) Expression of MYH10 in platelets of FPD/AML patients having a germline mutation of RUNX1

The findings that MYH10 is absent from platelets of normal healthy subjects and that it is directly negatively regulated by RUNX1 in MK suggested that MYH10 could be present in platelets of patients with genetic alteration of RUNX1.

To confirm this hypothesis we first focused on familial platelet disorder with predisposition to acute myeloid leukemia (FPD/AML, OMIM 601399). FPD/AML is an autosomal dominant disorder characterized by a dysmegakaryopoiesis, qualitative and quantitative platelet defects, and a propensity to develop MDS and/or AML. Importantly, several types of heterozygous germ-line mutations or deletions in *RUNX1,* including missense, frameshift and nonsense mutations, large intragenic deletion and single nucleotide deletion in the Runt domain have been identified.

We analyzed by Western Blot MYH10 expression in platelets of three pedigrees. Two patients AII-1 and AII-2 form pedigree with mutation R174Q, two patients BII-2, BIII-1 from pedigree with mutation R139X and one patient from pedigree with RUNX1 deletion (D) on one allele were investigated. Platelets from three healthy individuals (without RUNX1 alteration) (C1, C2 and C3) were used as negative controls.

The platelet-rich plasma (PRP) of patients and healthy individuals was prepared by centrifugation at 900 rpm for 10 minutes. Platelets were pelleted by centrifugation at 2000 rpm for 10 minutes. Remaining red cells (GPA⁺) were depleted using an immunomagnetic beads technique (MILTENYI, BIOTEC). For immunoblotting, lysates were obtained from 2x10⁶ platelets/µl, 30 µg of proteins were resolved by SDS-PAGE and transferred to nitrocellulose membranes. Blots were incubated with an antibody against MYH10 (CELL SIGNALLING) and reprobed with an antibody against HSC70 followed by HRP-linked secondary antibodies and ECL reagent for detection (AMERSHAM PHARMACIA BIOTECH).

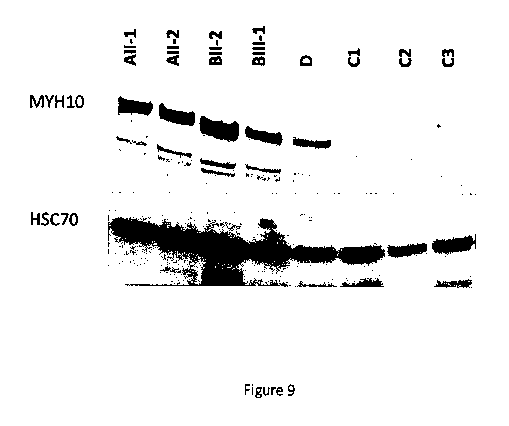

The figure 9 shows the MYH10 expression as compared to HSC70 in FPD/AML patients with Runx1 mutations (AII-1 AII-2, BII-2, BIII-1, and D) as compared to healthy individuals (without RUNX1 alteration) (C1, C2 and C3).

The results show that MYH10 was present in platelets of all five FPD/AML patients and absent from platelets of three healthy individuals (Figure 9).

### 9) Expression of MYH10 in platelets of CMML by Western blot

Then, we investigated the possibility to use the detection of MYH10 in platelets of CMML patients as a diagnostic of RUNX1 alterations.

It has been published, that these last ones are detected in up to 40% of chronic monomyelocytic leukemia.

Thus, we performed Western blot analysis of MYH10 in platelets of several patients, demonstrating that such approach is feasible. The Western blot analysis was done as described in paragraph above.

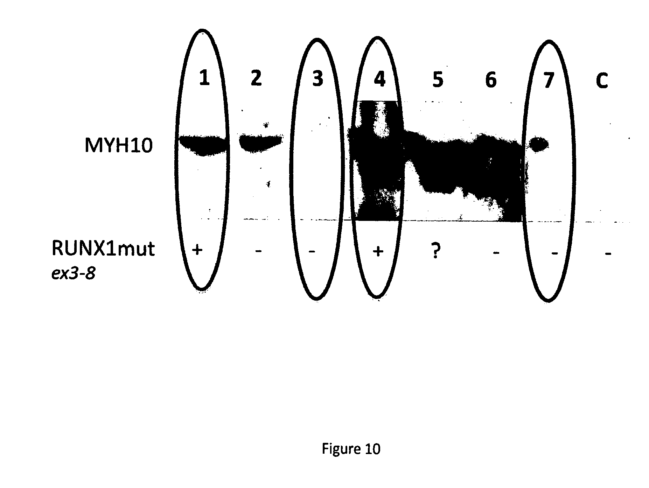

The figure 10 shows the Western blot analysis of MYH10 in platelets of CMML (1-7) patients and of one healthy individual used as a control.

The results show that such analysis enable the detection of MYH10 in platelets of CMML patients as a diagnostic of RUNX1 alterations.

### 10) Correlation between the presence of MYH10 in platelets of CMML patients and the genomic status of RUNX1

As shown in Figure 10, five from seven CMML samples were positive for MYH10. Two of them were found positive for RUNX1 mutations, one of them was not investigated and two of them were found negative for mutations in exons 3 to 8 of RUNX1. In these two patients, exons 1 and 2 will be sequenced.

Recently, a mutation in promoter 1 of RUNX1 in one pedigree FPD/AML was found (unpublished data) suggesting that this kind of mutation can be also found in CMML. Thus, in patients negative for RUNX1 alterations in exons 3-8 but positive for MYH10 in platelets, the exons 1 and 2 and the promoter will be sequenced.

Together, we investigated 21 CMML samples.

The results have shown that 13 of them were positive for MYH10 in platelets but except for six patients with known RUNX1 status (see Figure 10), other samples should be sequenced to perform the correlation between MYH10 presence in platelets and RUNX1 alterations.

### 11) MYH10 detection in platelets of patients with other thrombocytopenia of unknown origin.

MYH10 expression was also detected in platelets from one of two patients with unexplained thrombocytopenia.

These samples will be consequently sequenced for RUNX1.

SEQUENCE LISTING

<110>  Institut Gustave Roussy (IGR)
       Bluteau, Dominique
       Chang-Marchand, Yunchua
       Favier, Rémi
       Lordier, Larissa
       Raslova, Hana
       Vainchencker, William

<120>  MYH10 AS A NEW DIAGNOSTIC MARKER OF PATHOLOGIES RESULTING FROM
       RUNX1 INACTIVATION

<130>  IGR-B-0003-EP1

<160>  3

<170>  PatentIn version 3.5

<210>  1
<211>  1976
<212>  PRT
<213>  Homo sapiens

<400>  1

<210>  2
<211>  7619
<212>  DNA
<213>  Homo sapiens

<400>  2

actgaggcgc tggatctgtg gtcgcggctg gggacgtgcg cccgcgccac catcttcggc 60
tgaagaggca attgcttttg gatcgttcca tttacaatgg cgcagagaac tggactcgag 120
gatccagaga ggtatctctt tgtggacagg gctgtcatct acaaccctgc cactcaagct 180
gattggacag ctaaaaagct agtgtggatt ccatcagaac gccatggttt tgaggcagct 240
agtatcaaag aagaacgggg agatgaagtt atggtggagt tggcagagaa tggaaagaaa 300
gcaatggtca acaaagatga tattcagaag atgaacccac ctaagttttc caaggtggag 360
gatatggcag aattgacatg cttgaatgaa gcttccgttt tacataatct gaaggatcgc 420
tactattcag gactaatcta tacttattct ggactcttct gtgtagttat aaacccttac 480
aagaatcttc caatttactc tgagaatatt attgaaatgt acagagggaa gaagcgtcat 540
gagatgcctc cacacatcta tgctatatct gaatctgctt acagatgcat gcttcaagat 600
cgtgaggacc agtcaattct ttgcacgggt gagtcaggtg ctgggaagac agaaaataca 660
aagaaagtta ttcagtacct tgcccatgtt gcttcttcac ataaaggaag aaaggaccat 720
aatattcctg gggaacttga acggcagctt ttgcaagcaa atccaattct ggaatcattt 780
ggaaatgcga agactgtgaa aaatgataac tcatctcgtt ttggcaaatt tattcggatc 840
aactttgatg taactggcta tatcgttggg gccaacattg aaacatacct tctggaaaag 900
tctcgtgctg ttcgtcaagc aaaagatgaa cgtacttttc atatctttta ccagttgtta 960
tctggagcag gagaacacct aaagtctgat ttgcttcttg aaggatttaa taactacagg 1020
tttctctcca atggctatat tcctattccg ggacagcaag acaaagataa tttccaggag 1080
accatggaag caatgcacat aatgggcttc tcccatgaag agattctgtc aatgcttaaa 1140
gtagtatctt cagtgctaca gtttggaaat atttctttca aaaaggagag aaatactgat 1200
caagcttcca tgccagaaaa tacagttgcg cagaagctct gccatcttct tgggatgaat 1260
gtgatggagt ttactcgggc catcctgact ccccggatca aggtcggccg agactatgtg 1320
caaaaagccc agaccaaaga acaggcagat tttgcagtag aagcattggc aaaagctacc 1380
tatgagcggc tctttcgctg gctcgttcat cgcatcaata aagctctgga taggaccaaa 1440
cgtcagggag catctttcat tggaatcctg gatattgctg gatttgaaat ttttgagctg 1500
aactcctttg aacaactttg catcaactac accaatgaga agctgcagca gctgttcaac 1560
cacaccatgt ttatcctaga acaagaggaa taccagcgcg aaggcatcga gtggaacttc 1620
atcgatttcg ggctggatct gcagccatgc atcgacctaa tagagagacc tgcgaaccct 1680
cctggtgtac tggccctttt ggatgaagaa tgctggttcc ctaaagccac agataaaacc 1740
tttgttgaaa aactggttca agagcaaggt tcccactcca agtttcagaa acctcgacaa 1800
ttaaaagaca aagctgattt ttgcattata cattatgcag ggaaggtgga ctataaggca 1860
gatgagtggc tgatgaagaa tatggacccc ctgaatgaca acgtggccac ccttttgcac 1920
cagtcatcag acagatttgt ggcagagctt tggaaagatg tggaccgtat cgtgggtctg 1980
gatcaagtca ctggtatgac tgagacagct tttggctccg catataaaac caagaagggc 2040

atgtttcgta ccgttgggca actctacaaa gaatctctca ccaagctgat ggcaactctc 2100
cgaaacacca accctaactt tgttcgttgt atcattccaa atcacgagaa gagggctgga 2160
aaattggatc cacacctagt cctagatcag cttcgctgta atggtgtcct ggaagggatc 2220
cgaatctgtc gccagggctt ccctaaccga atagttttcc aggaattcag acagagatat 2280
gagatcctaa ctccaaatgc tattcctaaa ggttttatgg atggtaaaca ggcctgtgaa 2340
cgaatgatcc gggctttaga attggaccca aacttgtaca gaattggaca gagcaagata 2400
tttttcagag ctggagttct ggcacactta gaggaagaaa gagatttaaa aatcaccgat 2460
atcattatct tcttccaggc cgtttgcaga ggttacctgg ccagaaaggc ctttgccaag 2520
aagcagcagc aactaagtgc cttaaaggtc ttgcagcgga actgtgccgc gtacctgaaa 2580
ttacggcact ggcagtggtg gcgagtcttc acaaaggtga agccgcttct acaagtgact 2640
cgccaggagg aagaacttca ggccaaagat gaagagctgt tgaaggtgaa ggagaagcag 2700
acgaaggtgg aaggagagct ggaggagatg gagcggaagc accagcagct tttagaagag 2760
aagaatatcc ttgcagaaca actacaagca gagactgagc tctttgctga agcagaagag 2820
atgagggcaa gacttgctgc taaaaagcag gaattagaag agattctaca tgacttggag 2880
tctagggttg aagaagaaga agaaagaaac caaatcctcc aaaatgaaaa gaaaaaaatg 2940
caagcacata ttcaggacct ggaagaacag ctagacgagg aggaaggggc tcggcaaaag 3000
ctgcagctgg aaaaggtgac agcagaggcc aagatcaaga agatggaaga ggagattctg 3060
cttctcgagg accaaaattc caagttcatc aaagaaaaga aactcatgga agatcgcatt 3120
gctgagtgtt cctctcagct ggctgaagag gaagaaaagg cgaaaaactt ggccaaaatc 3180
aggaataagc aagaagtgat gatctcagat ttagaagaac gcttaaagaa ggaagaaaag 3240
actcgtcagg aactggaaaa ggccaaaaga aaactcgacg gggagacgac cgacctgcag 3300
gaccagatcg cagagctgca ggcgcagatt gatgagctca agctgcagct ggccaagaag 3360
gaggaggagc tgcagggcgc actggccaga ggtgatgatg aaacactcca taagaacaat 3420
gcccttaaag ttgtgcgaga gctacaagcc caaattgctg aacttcagga agactttgaa 3480
tccgagaagg cttcacggaa caaggccgaa aagcagaaaa gggacttgag tgaggaactg 3540
gaagctctga aaacagagct ggaggacacg ctggacacca cggcagccca gcaggaacta 3600
cgtacaaaac gtgaacaaga agtggcagag ctgaagaaag ctcttgagga ggaaactaag 3660
aaccatgaag ctcaaatcca ggacatgaga caaagacacg caacagccct ggaggagctc 3720
tcagagcagc tggaacaggc caagcggttc aaagcaaatc tagagaagaa caagcagggc 3780
ctggagacag ataacaagga gctggcgtgt gaggtgaagg tcctgcagca ggtcaaggct 3840
gagtctgagc acaagaggaa gaagctcgac gcgcaggtcc aggagctcca tgccaaggtc 3900
tctgaaggcg acaggctcag ggtggagctg gcggagaaag caagtaagct gcagaatgag 3960
ctagataatg tctccaccct tctggaagaa gcagagaaga agggtattaa atttgctaag 4020
gatgcagcta gtcttgagtc tcaactacag gatacacagg agcttcttca ggaggagaca 4080

cgccagaaac taaacctgag cagtcggatc cggcagctgg aagaggagaa gaacagtctt 4140
caggagcagc aggaggagga ggaggaggcc aggaagaacc tggagaagca agtgctggcc 4200
ctgcagtccc agttggctga taccaagaag aaagtagatg acgacctggg aacaattgaa 4260
agtctggaag aagccaagaa gaagcttctg aaggacgcgg aggccctgag ccagcgcctg 4320
gaggagaagg cactggcgta tgacaaactg gagaagacca agaaccgcct gcagcaggag 4380
ctggacgacc tcacggtgga cctggaccac cagcgccagg tcgcctccaa cttggagaag 4440
aagcagaaga agtttgacca gctgttagca gaagagaaga gcatctctgc tcgctatgcc 4500
gaagagcggg accgggccga agccgaggcc agagagaaag aaaccaaagc cctgtcactg 4560
gcccgggccc tcgaggaagc cctggaggcc aaggaggagt ttgagaggca gaacaagcag 4620
ctccgagcag acatggaaga cctcatgagc tccaaagatg atgtgggaaa aaacgttcac 4680
gaacttgaaa aatccaaacg ggccctagag cagcaggtgg aggaaatgag gacccagctg 4740
gaggagctgg aagacgaact ccaggccacg gaagatgcca agcttcgtct ggaggtcaac 4800
atgcaggcca tgaaggcgca gttcgagaga gacctgcaaa ccagggatga gcagaatgaa 4860
gagaagaagc ggctgctgat caaacaggtg cgggagctcg aggcggagct ggaggatgag 4920
aggaaacagc gggcgcttgc tgtagcttca aagaaaaaga tggagataga cctgaaggac 4980
ctcgaagccc aaatcgaggc tgcgaacaaa gctcgggatg aggtgattaa gcagctccgc 5040
aagctccagg ctcagatgaa ggattaccaa cgtgaattag aagaagctcg tgcatccaga 5100
gatgagattt ttgctcaatc caaagagagt gaaaagaaat tgaagagtct ggaagcagaa 5160
atccttcaat tgcaggagga acttgcctca tctgagcgag cccgccgaca cgccgagcag 5220
gagagagatg agctggcgga cgagatcacc aacagcgcct ctggcaagtc cgcgctgctg 5280
gatgagaagc ggcgtctgga agctcggatc gcacagctgg aggaggagct ggaagaggag 5340
cagagcaaca tggagctgct caacgaccgc ttccgcaaga ccactctaca ggtggacaca 5400
ctgaacgccg agctagcagc cgagcgcagc gccgcccaga agagtgacaa tgcacgccag 5460
caactggagc ggcagaacaa ggagctgaag gccaagctgc aggaactcga gggtgctgtc 5520
aagtctaagt tcaaggccac catctcagcc ctggaggcca agattgggca gctggaggag 5580
cagcttgagc aggaagccaa ggaacgagca gccgccaaca aattagtccg tcgcactgag 5640
aagaagctga aagaaatctt catgcaggtt gaggatgagc gtcgacacgc ggaccagtat 5700
aaagagcaga tggagaaggc caacgctcgg atgaagcagc ttaaacgcca gctggaggaa 5760
gcagaagaag aagcgacgcg tgccaacgca tctcggcgta aactccagcg ggaactggat 5820
gatgccaccg aggccaacga gggcctgagc cgcgaggtca gcaccctgaa gaaccggctg 5880
aggcggggtg gccccatcag cttctcttcc agccgatctg gccggcgcca gctgcacctt 5940
gaaggagctt ccctggagct ctccgacgat gacacagaaa gtaagaccag tgatgtcaac 6000
gagacgcagc caccccagtc agagtaaagt tgcaggaagc cagaggaggc aatacagtgg 6060
gacagttagg aatgcacccg gggcctcctg cagatttcgg aaattggcaa gctacgggat 6120

tccttcctga aagatcaact gtgtcttaag gctctccagc ctatgcatac tgtatcctgc 6180
ttcagactta ggtacaattg ctcccctttt tatatataga cacacacagg acacatatat 6240
taaacagatt gtttcatcat tgcatctatt ttccatatag tcatcaagag accattttat 6300
aaaacatggt aagacccttt ttaaaacaaa ctccaggccc ttggttgcgg gtcgctgggt 6360
tattggggca gcgccgtggt cgtcactcag tcgctctgca tgctctctgt catacagaca 6420
ggtaacctag ttctgtgttc acgtggcccc cgactcctca gccacatcaa gtctcctaga 6480
ccactgtgga ctctaaactg cacttgtctc tctcatttcc ttcaaataat gatcaatgct 6540
atttcagtga gcaaactgtg aaaggggctt tggaaagagt aggaggggtg ggctggatcg 6600
gaagcaacac ccatttgggg ttaccatgtc catcccccaa ggggggccct gcccctcgag 6660
tcgatggtgt cccgcatcta ctcatgtgaa ctggccttgg cgagggctgg tctgtgcata 6720
gaagggatag tggccacact gcagctgagg ccccaggtgg cagccatgga tcatgtagac 6780
ttccagatgg tctcccgaac cgcctggctc tgccggcgcc ctcctcacgt caggagcaag 6840
cagccgtgga cccctaagcc gagctggtgg aaggcccctc cctgtcgcca gccgggccct 6900
catgctgacc ttgcaaattc agccgctgct ttgagcccaa aatgggaata ttggttttgt 6960
gtccgaggct tgttccaagt ttgtcaatga ggtttatgga gcctccagaa cagatgccat 7020
cttcctgaat gttgacatgc cagtgggtgt gactccttca tttttccttc tcccttccct 7080
ttggacagtg ttacagtgaa cacttagcat cctgtttttg gttggtagtt aagcaaactg 7140
acattacgga aagtgcctta gacactacag tactaagaca atgttgaata tatcattcgc 7200
ctctataaca atttaatgta ttcagttttg actgtgcttc atatcatgta cctctctagt 7260
caaagtggta ttacagacat tcagtgacaa tgaatcagtg ttaattctaa atccttgatc 7320
ctctgcaatg tgcttgaaaa cacaaacctt ttgggttaaa agctttaaca tctattagga 7380
agaatttgtc ctgtgggttt ggaatcttgg attttccccc tttatgaact gtactggctg 7440
ttgaccacca gacacctgac cgcaaatatc ttttcttgta ttcccatatt tctagacaat 7500
gatttttgta agacaataaa tttattcatt atagatattt gcgcctgctc tgtttacttg 7560
aagaaaaaag cacccgtgga gaataaagag acctcaataa acaagaataa tcatgtgaa 7619

<210> 3
<211> 480
<212> PRT
<213> Homo sapiens

<400> 3

## Claims

1. A method of diagnosing a pathology resulting from a runt-related transcription factor 1 (RUNX1) inactivation in a subject, which comprises the step of i) determining the platelets' myosin non-muscle heavy chain 10 (MYH10) expression level in a biological sample from said subject, and wherein a detectable platelets' MYH10 expression level is indicative of a pathology resulting from a runt-related transcription factor 1 (RUNX1) inactivation.

2. The method of claim 1, wherein said subject is a human.

3. The method of claim 1, wherein said pathology resulting from a runt-related transcription factor 1 (RUNX1) is selected in the group comprising familial platelet disorder with propensity to acute myeloid leukaemia (FPD/AML), acute myeloid leukaemia (AML) and chronic myelomonocytic leukemia (CMML).

4. The method of claim 3, wherein said pathology resulting from a runt-related transcription factor 1 (RUNX1) is FPD/AML.

5. The method of claim 1, wherein said biological sample is a blood sample.

6. The method of claim 1, wherein said method is for testing a subject thought to develop or to be predisposed to developing familial platelet disorder with propensity to acute myeloid leukaemia (FPD/AML), other unexplained thrombocytopenia linked to RUNX1 pathway deregulation, to acute myeloid leukaemia (AML) and/or to chronic myelomonocytic leukemia (CMML).

7. The method of claim 1, wherein said method further comprises the step (ii) of comparing said platelets' MYH10 expression level in said biological sample with a control.

8. The method of claim 7, wherein said control corresponds to the MYH10 expression level in a control sample corresponding to platelets of a biological sample from a healthy subject.

9. The method of claim 1, wherein said step (i) is done by determining the platelets' MYH10 protein (SEQ ID n°1) expression level expression in said biological sample.

10. A kit for diagnosing a pathology resulting from a runt-related transcription factor 1 (RUNX1) inactivation in a subject, which comprises i) at least one antibody for determining the platelet MYH10 expression level in a biological sample from said subject.

11. The kit of claim 10 further comprising ii) at least one mean for purifying platelets from the biological sample.

12. The kit of claim 11, wherein said mean is for purifying platelets from a blood sample.
